(19) Europäisches Patentamt
European Patent Office
Office européen des brevets

(11) **EP 3 492 911 B1**

(12) **EUROPÄISCHE PATENTSCHRIFT**

(45) Veröffentlichungstag und Bekanntmachung des Hinweises auf die Patenterteilung:
**16.04.2025  Patentblatt 2025/16**

(21) Anmeldenummer: **17208482.4**

(22) Anmeldetag: **19.12.2017**

(51) Internationale Patentklassifikation (IPC):
**G01N 22/00** (2006.01)    **G01N 33/38** (2006.01)

(52) Gemeinsame Patentklassifikation (CPC):
**G01N 22/00; G01N 33/386; G01N 33/388**

(54) **VERFAHREN ZUR BESTIMMUNG DER KONZENTRATION EINER ZUSATZKOMPONENTE IN EINEM KÖRPER AUS KERAMISCHEM ODER GLASIGEM WERKSTOFF**

METHOD FOR DETERMINING THE CONCENTRATION OF AN ADDITIONAL COMPONENT IN A BODY MADE FROM CERAMIC OR GLASSY MATERIAL

PROCÉDÉ DE DÉTERMINATION DE LA CONCENTRATION D'UN COMPOSANT SUPPLÉMENTAIRE DANS UN CORPS EN MATÉRIAU CÉRAMIQUE OU VITREUX

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität:  **29.11.2017  EP 17204532**

(43) Veröffentlichungstag der Anmeldung:
**05.06.2019  Patentblatt 2019/23**

(73) Patentinhaber: **Heraeus Quarzglas GmbH & Co. KG**
**63450 Hanau (DE)**

(72) Erfinder: **FRIEDRICH, Helmut**
**64807 Dieburg (DE)**

(74) Vertreter: **Gille Hrabal Partnerschaftsgesellschaft mbB**
**Patentanwälte**
**Brucknerstraße 20**
**40593 Düsseldorf (DE)**

(56) Entgegenhaltungen:
DE-A1- 102013 225 306      JP-A- S6 020 138
US-A1- 2017 031 006

EP 3 492 911 B1

**Beschreibung**

Technischer Hintergrund

Die Erfindung betrifft ein Verfahren zur Bestimmung der Konzentration einer Zusatzkomponente in einem Körperaus glasigem Werkstoff, umfassend die Messung der optischen Weglänge oder der Signallaufzeit einer den Körper aus glasigem Werkstoff in einer Messrichtung durchdringenden Messwelle.

Die chemische Natur des Körpers ausglasigem Werkstoff hängt von seinem Einsatzzweck ab. Er besteht beispielsweise teilweise oder vollständig aus einem Glas, insbesondere aus Quarzglas. Unter Quarzglas wird hier dotiertes oder undotiertes Kieselglas mit einem $SiO_2$-Gehalt von mindestens 85% verstanden. Das Glas ist porös, transparent, opak, farbig, insbesondere kann es auch schwarz sein.

Auch die geometrische Form des Körpers aus glasigem Werkstoff richtet sich nach dem Einsatzzweck. Er dient beispielsweise als Bauteil oder als Halbzeug für die Herstellung optischer Fasern, bei der Lampenfertigung, im chemischen Apparatebau oder in der Halbleiterfertigung, und er liegt beispielsweise in Form von Rohren, Vollzylindern, Platten, Flanschen, Ringen, Blöcken, Kolben, Abdeckplatten, Reflektoren, Reflektorträgern, Spiegelsubstratrohlingen, Linsen Trägerhorden, Glocken, Tiegeln, Schutzschilden, Reaktoren und Apparaturen vor.

Die physikalischen und chemischen Eigenschaften des Werkstoffs werden durch unerwünschte Verunreinigungen und durch absichtlich zugesetzte Dotierstoffe beeinflusst. Derartige Verunreinigungen und Dotierstoffe werden im Folgenden auch als "Zusatzkomponenten" bezeichnet. Zusatzkomponenten in Quarzglas sind beispielsweise Hydroxylgruppen (OH), Chlor (Cl), Fluor (F) und metallische Elemente (Ge, B, P, Al, Ti, Fe usw.). Hydroxylgruppen erzeugen beispielsweise eine Absorptionsbande im Infrarotbereich, können aber auch die Strahlenbeständigkeit von Quarzglas gegenüber UV-Strahlung verbessern. Eine Dotierung von Quarzglas mit Titandioxid bis zu 8 Gew.-% verringert den thermischen Ausdehnungskoeffizienten.

Eine besonders wichtige Eigenschaft derartiger Körper aus glasigem Werkstoff ist ihre Brechzahl und deren räumliche Verteilung. So bestimmt beispielsweise das radiale Brechzahlprofil einer Faser-Vorform die Wellenleitereigenschaften der daraus gezogenen optischen Faser. Viele Zusatzkomponenten wirken sich auf die Brechzahl aus. Fluor ist ein Dotierstoff, der die Brechzahl von Quarzglas verringert.

Stand der Technik

Zur Charakterisierung von Glasmaterialien und insbesondere zur Ermittlung der Konzentration einer Zusatzkomponente in Körpern aus glasigem Werkstoff werden häufig spektroskopische Methoden angewandt, wie etwa die Infrarot- oder die Raman-Spektroskopie. Dabei werden Absorption beziehungsweise Streuung elektromagnetischer Messwellen in oder an einer Messprobe ausgewertet. Besonders Raman-Messungen erfordern einen hohen Justage-Aufwand. Besonders nachteilig ist aber, dass in der Regel Messproben bestimmter Größe aus dem Körper aus glasigem Werkstoff entnommen und aufwändig vorbereitet werden müssen.

Diesen Nachteil vermeidet eine zerstörungsfreie Messmethode, die Schall- oder Ultraschallwellen einsetzt. Die Ermittlung der Konzentration der Zusatzkomponente beruht hierbei auf Auswertung der Überlagerung von solchen Schallwellen, die den Körper aus glasigem Werkstoff mindestens teilweise durchdringen mit anderen Schallwellen, die den Körper ausglasigem Werkstoff nicht oder auf kürzerer Strecke durchdringen. Ein derartiges

Messverfahren ist beschrieben im Fachartikel von Wei, Ting-Cun "Acoustic properties of silica glass doped with fluorine", Journal of Non-Crystalline Solids 321 (2003), S. 126-133.

Dabei werden Quarzglasproben mit verschiedenen Fluor-Konzentrationen mittels Ultraschallmessungen (LSAW; leaky surface acoustic wave) untersucht, wobei sich zeigt, dass die Ausbreitungsgeschwindigkeit der Ultraschallwellen in dem Quarzglaskörper mit steigender Fluorkonzentration linear abnimmt. Die Messung oder Berechnung der Ausbreitungsgeschwindigkeit der Ultraschallwellen Quarzglaskörper ermöglicht daher die Ermittlung der Fluor-Konzentration. Die Ausbreitungsgeschwindigkeit der Ultraschallwellen wird anhand der Interferenz gepulster Tonburst-Signale ohne und mit Durchschallung der Messprobe ermittelt. Zum Einkoppeln der Ultraschallwellen vom Ultraschallwandler in den Messkörper wird Wasser als Kopplungsmedium eingesetzt.

Die US 2017/031006 A1 beschreibt eine optische Methode zur Erfassung von Schäden, Materialveränderungen oder Verschmutzungen an einer Turbinenschaufel und anderen Bauteilen aus Metall- oder Keramik. Dazu wird die Phasen-und/oder die Amplitudenänderung eines reflektierten Messstrahls gegenüber einem vom Werkstück unbeeinflussten Vergleichsstrahl mittels "Vektornetzwerkanalyse" ausgewertet. Der Frequenzbereich des Messstrahls liegt im Radiofrequenz-, HF-Frequenz- oder Mikrowellenfrequenzbereich.

DE 10 2013 225306 A1 offenbart ein Verfahren zur Bestimmung der Konzentration einer Zusatzkomponente in einem Körper aus Keramikpartikeln mittels Mikrowellenstrahlung.

## Technische Aufgabenstellung

Grundsätzlich können per Ultraschallmessung auch großvolumige Körper aus glasigem Werkstoff zerstörungsfrei vermessen werden. Zum Einkoppeln der Ultraschallwellen ist aber eine aufwändige Einkoppeltechnik erforderlich oder mindestens ein Kopplungsmedium, beispielsweise ein den Glaskörper umgebendes Wasserbad. Dies verhindert eine Vermessung während des Produktionsprozesses oder erschwert sie jedenfalls.

Der Erfindung liegt daher die Aufgabe zugrunde, eine zerstörungsfreie Methode zur Ermittlung einer Konzentration einer Zusatzkomponente in einem Körper ausglasigem Werkstoff anzugeben, das auch für eine Messung im Produktionsprozess des betreffenden Körpers geeignet ist.

## Zusammenfassung der Erfindung

Diese Aufgabe wird durch das im Anspruch 1 beanspruchte Verfahren gelöst.

Die Gigahertz-Strahlung im Sinne der Erfindung umfasst den Frequenzbereich zwischen 20 bis 300 Gigahertz (GHz). Diese Strahlung ist in der Lage, einen Körper aus glasigem Werkstoff unabhängig von seiner chemischen Natur und seiner inneren Struktur, wie Porosität, Dichte und Farbe zu durchdringen, ohne dass sie dabei eine Veränderung der Materialeigenschaften bewirkt. Die Strahlung selbst erfährt aber in dem Glas eine Veränderung, die als Laufzeitveränderung eines Messstrahls gegenüber dem vom Werkstoff unbeeinflussten Vergleichsstrahl ermittelbar ist. Die Laufzeitveränderung ergibt sich beispielsweise dadurch, dass der Werkstoff eine magneto-optische, elektro-optische, thermo-optische, oder chemooptische Eigenschaft besitzt, durch die die einfallende Gigahertz-Strahlung veränderbar ist und sich als Laufzeitveränderung äußert. Es hat sich gezeigt, dass die Laufzeitveränderung insbesondere von der chemischen Zusammensetzung des Werkstoffs abhängt, so dass die Konzentration eines jeden Inhaltsstoffes bei ansonsten gleichbleibender Struktur und Zusammensetzung des Werkstoffs ermittelt werden kann.

Beim erfindungsgemäßen Verfahren wird somit die Laufzeitdifferenz von Messwellen ermittelt, ähnlich wie bei den Ultraschallwellen-Messungen, wobei aber im Unterschied dazu auf ein Einkoppelmedium verzichtet werden kann. Dies ermöglicht ein zerstörungsfreies Vermessen des Körpers aus glasigem Werkstoff auch während seines Herstellungsprozesses.

Durch den Einsatz von modulierter Gigahertz-Strahlung verringert sich der Aufwand für die Probenvorbereitung, und die Notwendigkeit einer Probenentnahme entfällt. Somit ist der Mess- und Auswerteaufwand gering im Vergleich zu den eingangs genannten spektroskopischen Analysemethoden.

Das erfindungsgemäße Verfahren vereint daher die Auswertung von Laufzeitdifferenzen aus der bekannten Ultraschall-Messung, setzt aber keine Schallwellen ein, sondern elektromagnetische Strahlung, wie bei spektroskopischen Analysemethoden. Es vermeidet daher die jeweiligen Nachteile der bekannten Methoden, insbesondere hinsichtlich Probenaufbereitung und der Notwendigkeit des Einsatzes von Einkoppelmedien.

Bei dem erfindungsgemäßen Verfahren ist ein Sender oder es sind mehrere Sender für die Aussendung der Gigahertz-Strahlung vorgesehen, die in der Regel eine Strahloptik passiert, mittels der die Gigahertz-Strahlung auf den Körper aus glasigem Werkstoff gerichtet wird. Die Gigahertz-Strahlung wird von dem Körper aus glasigem Werkstoff und/oder von einem gegenüberliegend zum Sender in Strahlungsrichtung angeordneten Reflektor reflektiert, und die reflektierte Strahlung wird von mindestens einem Empfänger empfangen. Anstelle der Vermessung reflektierter Strahlung kann sich auch ein Sender-Empfänger-Paar am zu vermessenden Körper aus glasigem Werkstoff gegenüberliegen, so dass die vom Körper transmittierte Gigahertz-Strahlung direkt gemessen wird.

Die Laufzeiten können gemessen werden, indem Strahlungspulse verwendet werden, die sich zeitlich "lokalisieren lassen", oder indem Dauerstrichstrahlung (CW-Strahlung) durch Modulation ein Zeitstempel aufgeprägt wird. Der Zeitpunkt des im Empfänger empfangenen Messsignals wird an eine Auswerteeinrichtung übermittelt, die die Laufzeit ermittelt. Die Laufzeit von Gigahertz-Strahlung, die den zu vermessenden Körper aus glasigem Werkstoff passiert, wird verglichen mit der Laufzeit von Strahlung die den Körper nicht passiert. Die so ermittelte, durch das Material des Körpers aus glasigem Werkstoff verursachte Laufzeitänderung ist ein Maß für die Konzentration der Zusatzkomponente. Aus dem Vergleich der Laufzeiten ergibt sich somit die Konzentration der Zusatzkomponente. Durch die Modulation werden der CW-Strahlung Markierungen für die Laufzeitbestimmung aufgeprägt.

Die den Körper nicht passierende Gigahertz-Strahlung kann beispielsweise Strahlung sein, die am Körper reflektiert wird oder Gigahertz-Strahlung, in deren Strahlengang der Körper aus glasigem Werkstoff nicht positioniert ist oder Gigahertz-Strahlung, deren Ausbreitungsgeschwindigkeit bekannt ist, so dass allein aus der Kenntnis der Länge der freien Laufstrecke und des Füllmediums (beispielsweise Luft), die Laufzeit auch ohne erneute Messung ermittelt werden kann.

Bei dieser Verfahrensvariante sind somit die Entfernung zwischen Sender und gegenüberliegendem Empfänger beziehungsweise zwischen Sender- und Empfängereinheit und Reflektor bekannt, sodass anhand der bekannten Ausbreitungsgeschwindigkeit der Gigahertz-Strahlung (in Luft) die durch das Material des Körpers aus glasigem Werkstoff verursachte Veränderung der Ausbreitungsgeschwindigkeit ermittelt werden kann.

Sender und Empfänger können in einem Bauteil zusammengefasst sein und dabei praktisch ortsgleich angeordnet sein, sodass sie jederzeit den gleichen Abstand zum Körper aus glasigem Werkstoff haben, was die Laufzeitmessung vereinfacht. Ein derartiges Bauteil wird als "Transceiver" bezeichnet.

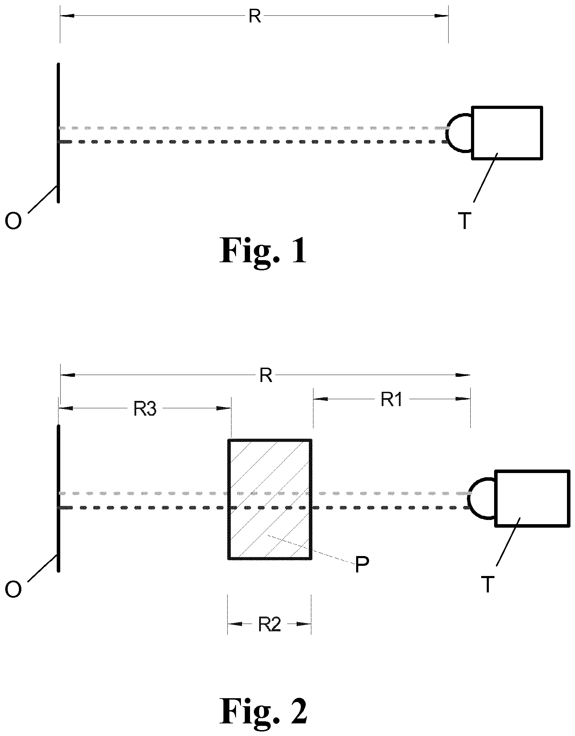

Der Fall der unbeeinflussten Rückreflexion elektromagnetischer Strahlung an einem reflektierenden Objekt bei Positionsgleichheit von Sender und Empfänger (Transceiver) ist schematisch in **Figur 1** dargestellt. Die von der Sendeeinheit des Transceivers T abgegebene elektromagnetische Strahlung durchläuft die freie Luftstrecke zum reflektierenden Objekt O zweimal, bis sie von der Empfangseinheit des Transceivers T wieder erfasst wird. Zwischen der Laufzeit t und der Entfernung R zum reflektierenden Objekt O gilt dann allgemein folgender Zusammenhang:

$$R \quad = \quad c_0 \times t \,/\, 2 \qquad\qquad (1)$$

Mit:

R = Entfernung zwischen Transceiver und Reflektor
$c_0$ = Lichtgeschwindigkeit an Luft [m/s]
t = Laufzeit [s]

**Figur 2** zeigt schematisch den Fall, dass sich im Strahlengang eine Messprobe P befindet, die die Ausbreitungsgeschwindigkeit der elektromagnetischen Strahlung verändert. Gegenüber der in Gleichung (1) verwendeten Laufzeit t ergibt sich eine Laufzeitveränderung $|\Delta t_p|$ aus der sich die Ausbreitungsgeschwindigkeit $c_p$ der elektromagnetischen Strahlung in der Probe nach Gleichung (2) berechnen lässt.

$$c_p = R2/(R2/c_0 + \Delta tp) \qquad\qquad (2)$$

Mit:

R1, R3 = Strecken an Luft
R2 = Probenabmessung
$c_p$ = Strahlungs-Ausbreitungsgeschwindigkeit in der Probe
$|\Delta t_p|$ = Laufzeitveränderung

Die Ausbreitungsgeschwindigkeit $c_p$ ist ein Maß für den Werkstoff der Probe und insbesondere für deren Materialkonstanten, die von der chemischen Zusammensetzung und der Dichte abhängen.

Die Strahlungsoptik umfasst beispielsweise eine oder mehrere Linsen, die von dem mindestens einen Sender ausgesandte Gigahertz-Strahlung auf eine Mittelachse des zu vermessenden Körpers aus glasigem Werkstoff fokussieren. Dadurch können Körper aus glasigem Werkstoff mit unterschiedlichen seitlichen Abmessungen vermessen werden, ohne dass es eine Nachführung der Messvorrichtung oder der Strahlungsoptik bedarf.

Alternativ dazu kann die Strahlungsoptik auch einen Kollimator umfassen, wobei die vom Sender abgegebene Gigahertz-Strahlung als Parallelstrahl durch den Körper aus glasigem Werkstoff geleitet wird. Dies hat den Vorteil, dass Positionsveränderungen des Körpers aus glasigem Werkstoff senkrecht zur Strahlrichtung wenig Einfluss auf das Messergebnis haben.

Das erfindungsgemäße Verfahren ist einsetzbar für das Vermessen eines Körpers aus einem hochkieselsäurehaltigen Glas, insbesondere aus Quarzglas, wobei die Zusatzkomponente Hydroxylgruppen, Wasserstoff, ein Metalloxid und/oder ein Halogen umfasst, und wobei die Zusatzkomponente vorzugsweise Fluor ist.

Der Quarzglas-Körper kann dabei insbesondere auch als sogenannter "Sootkörper" aus porösem leichtverdichtetem $SiO_2$-Ruß vorliegen, der ein Halbzeug zur Herstellung von Vorformen für optische Fasern oder andere Produkte aus hochreinem, synthetischem Quarzglas darstellt. Auch Körper aus glasigem Werkstoff, die sichtbares Licht oder Infrarotstrahlung absorbieren, sind mittels des erfindungsgemäßen Verfahrens vermessbar, da die Gigahertz-Strahlung in der Lage ist, derartige Körper in einer für die Messung ausreichend großen Schichtdicke zu durchdringen.

Dies gilt gleichermaßen für opakes, porenhaltiges Glas, insbesondere opakes Quarzglas, sowie für titanhaltiges Glas, das für die Herstellung von Spiegelsubstrohlingen mit geringem thermischem Ausdehnungskoeffizienten verwendet wird.

Die Modulation der Gigahertz-Strahlung erfolgt beispielsweise durch Modulation der Phase und/oder der Amplitude und/oder der Frequenz und/oder der effektive Wellenlänge und/oder der Wellenfront. Besonders bevorzugt ist die Gigahertz-Strahlung jedoch periodisch frequenzmoduliert; beispielsweise und bevorzugt wird frequenzmodulierte Dauerstrichradar eingesetzt, sogenannte FMCW-Strahlung.

Die periodische Frequenzmodulation kann einen Frequenz-Burst oder mehrere Frequenz-Bursts umfassen.

Dabei kann ein vorgegebener Frequenzbereich ein-oder mehrmals durchfahren werden.

Vorzugsweise wird der Körper aus glasigem Werkstoff aus unterschiedlichen Messrichtungen von der Gigahertz-Strahlung durchdrungen, wobei für jede Messrichtung eine jeweilige optische Weglänge oder eine jeweilige Signallaufzeit ermittelt wird.

Dabei wird der Körper aus glasigem Werkstoff von einem oder von mehreren Sendern in unterschiedlichen Richtungen mit der Gigahertz-Strahlung durchsetzt, die von einem oder von mehreren Empfängern empfangen und in elektrische Signale umgesetzt wird, wobei die Laufzeiten der Gigahertz-Strahlung gemessen werden und wobei aus den Laufzeiten Gigahertz-Strahlung eine räumliche Verteilung der Konzentration der Zusatzkomponente erhalten werden.

Sender und Empfänger können beispielsweise einander gegenüberliegend angeordnet sein. Es können auch mehrere Sender und Empfänger um den Umfang des Körpers aus glasigem Werkstoff herum paarweise angeordnet sein. Jedes Paar aus Sender und Empfänger kann als Transceiver ausgebildet sein, wobei mehrere Transceiver um den Umfang des zu vermessenden Körpers aus glasigem Werkstoff verteilt sind.

Der Körper aus glasigem Werkstoff kann relativ zum Empfänger-Senderpaar entlang seiner Mittelachse rotiert werden. Bei einer bevorzugten Verfahrensvariante ist der Körper aus glasigem Werkstoff aber ortsfest in Bezug auf mindestens einen Sender für die Gigahertz-Strahlung positioniert, wobei der Sender zur Ermittlung eines dreidimensionalen Profils der Zusatzkomponenten-Konzentration um den Körper aus glasigem Werkstoff bewegt wird.

Alternativ und besonders bevorzugt wird jedoch mindestens ein Paar aus einem Empfänger und einem Sender während eines Messvorgangs um die Längsachse oder eine virtuelle Mittelachse des Körpers aus glasigem Werkstoff rotiert. Bei dem Paar von Sender und Empfänger kann es sich wiederum um einen Transceiver handeln. Durch das Rotieren um den zu vermessenden Körper aus glasigem Werkstoff können mehrere Paare von Sendern und Empfängern simuliert werden. Auf diese Weise kann die Konzentrationsbestimmung der Zusatzkomponente ortsaufgelöst erfolgen.

Der Bewegung des Senders um eine virtuelle Mittelachse um den Körper aus glasigem Werkstoff folgt in der Regel auch die Strahlungsoptik. Der Reflektor kann dabei so ausgebildet sein, dass er den Körper aus glasigem Werkstoff vollständig umgibt, so dass in dem Fall eine Bewegung des Reflektors um die Rotationsachse nicht erforderlich ist.

Um Mehrfachreflexionen zu vermeiden, die das Messergebnis stören können, ist der Abstand zwischen der Oberfläche des Körpers aus glasigem Werkstoff und dem Sender beziehungsweise Empfänger vorzugsweise größer oder kleiner als die in der Hauptabstrahlrichtung der Gigahertz-Strahlung liegende Abmessung des Körpers.

Ausführungsbeispiel

Nachfolgend wird die Erfindung anhand eines Ausführungsbeispiels und einer Zeichnung näher erläutert. In der Zeichnung zeigt im Einzelnen

**Figur 1**   eine Skizze zur Ermittlung einer Entfernung zwischen einem Transceiver für Gigahertz-Strahlung und einem Reflektor

**Figur 2**   die Skizze von Figur 1 mit einer Messprobe im Strahlengang der elektromagnetischen Strahlung,

**Figur 3**   eine Vorrichtung zum räumlichen Vermessen eines zylinderförmigen Körpers mit Dauerstrich-Radarstrahlung in schematischer Darstellung, wobei in (b) eine Längsansicht, in (a) eine Vorderansicht und in (c) eine Rückansicht dargestellt sind,

**Figur 4**   ein Diagramm mit Brechzahlprofilen bei elektromagnetischer Strahlung einer Frequenz von 80 GHz, die anhand einer Vermessung eines zylinderförmigen Quarzglaskörpers mit Dauerstrich-Radarstrahlung ermittelten worden sind, und

**Figur 5**   ein Diagramm, das die über die Wanddicke eines Hohlzylinders gemittelte Fluor-Konzentration in einem Quarzglaskörper in Abhängigkeit von einer mittleren Brechzahl bei elektromagnetischer Strahlung einer Frequenz von 80 GHz zeigt.

Das erfindungsgemäße Verfahren dient zur Ermittlung der Konzentration einer Zusatzkomponente in einem Körper aus glasigem Werkstoff, insbesondere einem zylinderförmigen optischen Gegenstand, wie zum Beispiel einer Vorform für optische Fasern aus Quarzglas.

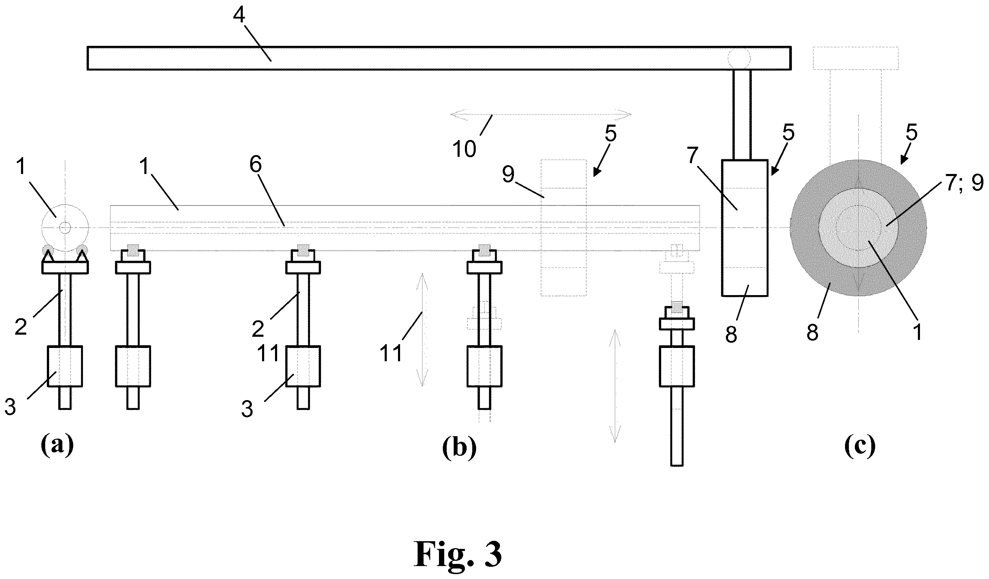

**Figur 3** zeigt schematisch eine Ausführungsform einer Vorrichtung, die zur Durchführung des erfindungsgemäßen Verfahrens geeignet ist. Der Körper aus glasigem Werkstoff liegt hierbei in Form eines Hohlzylinders 1 aus Quarzglas vor. Dieser ruht auf mehreren über die Länge gleichmäßig verteilten Probeauflagen 2, die jeweils mit Hub- und Senkeinrichtungen 3 ausgestattet sind, mittels denen die Probenauflage 2 vertikal nach oben in eine Stützposition und nach unten in eine Freigabeposition verfahrbar ist, wie von den Richtungspfeilen 11 angedeutet.

Oberhalb des Hohlzylinders 1 erstreckt sich eine Linearführung 4 für ein handelsübliches Radarmessgerät 5, das als kreisringförmiges Bauteil ausgeführt ist. Der Kreisring 8 umgibt eine Mittenöffnung 7, die größer ist als der Außendurchmesser des Hohlzylinders 1. Das Radarmessgerät 5 ist an der Linearführung 4 so montiert, dass die Mittenachse der Mittenöffnung 7 und die Längsachse 6 des Hohlzylinders 1 koaxial zu einander verlaufen. Daher kann das Radarmessgerät 5 mittels der Linearführung 4 entlang der Längsachse 6 des Hohlzylinders 1 bewegt werden.

Das Radargerät 5 umfasst einen Sender für die Abgabe frequenzmodulierter Radarstrahlung mit einer Frequenz von 80 GHz und einen Empfänger für diese Strahlung, die in Form eines Transceivers zusammengefasst sind. Der Transceiver ist innerhalb des Kreisrings 8 um die Mittenachse entlang einer Kreisbahn zusammen mit einer Strahlungsoptik verfahrbar, wie vom Richtungspfeil 10 angedeutet. Die Strahlungsoptik dient dazu, den Radarstrahl auf die Mittenachse und damit auch auf die Hohlzylinder-Längsachse 6 zu fokussieren. Die Breite des Ringspalts 9 zwischen dem Transceiver (Kreisring 8) und dem Außenmantel des Hohlzylinders 1 ist bekannt. Der Sender und der Empfänger sind mit einer (nicht dargestellten) Auswerte- und Steuereinrichtung verbunden.

Zur Ermittlung der Fluorkonzentration in dem Quarzglas des zu vermessenden Hohlzylinders 1 werden mittels des Radargeräts 5 die Laufzeiten der Radarstrahlung ermittelt. Die Ausbreitungsgeschwindigkeit der vom Sender emittierten Radarstrahlung wird je nach Materialkonstanten des Quarzglases, die einen Einfluss auf den Brechungsindex besitzen, unterschiedlich verzögert.

Um den Einfluss anderer Materialkonstanten auszuschließen, die bei der Fluor-Konzentrationsmessung stören würden, wird eine Eichmessung an einem Standard-Körper aus Quarzglas durchgeführt, bei dem die Fluor-Konzentration bekannt ist und das sich ansonsten nicht von dem Quarzglas des zu vermessenden Hohlzylinders 1 unterscheidet.

Die unter Einsatz des Standard-Körpers ermittelte Ausbreitungsgeschwindigkeit (beziehungsweise die Laufzeit der Radarwellen) kann in einfacher Weise mit der entsprechenden Laufzeitmessung unter Einsatz des zu vermessenden Hohlzylinders 1 und gegebenenfalls mit einer Laufzeitmessung ohne durchstrahlte Probe verglichen werden.

Der Außendurchmesser des Hohlzylinders 1 wird mit einer Genauigkeit mit einer Standardabweichung von weniger als 10 $\mu$m ermittelt und in die Auswerte- und Steuereinrichtung eingegeben. Im Ausführungsbeispiel beträgt der über die Länge gemittelte Außendurchmesser 201,60 mm, und die Weite des Ringspalts 9 beträgt 24,2 mm.

Die Wandstärke des Hohlzylinders 1 kann mittels allgemein bekannter taktiler oder optischer Messverfahren ermittelt werden. Besonders elegant ist aber eine Messmethode, bei der die Wandstärke (R2) des Hohlzylinder mit derselben Radar-Messtechnik bestimmt wird, indem die von einem Reflektor auf den Empfänger zurückreflektierten Reflexe der Radarstrahlung von Zylinderinnenwandung und Zylinderaußenwandung (mit und ohne Probe) ausgewertet werden. Die Auswerte- und Steuereinrichtung ermittelt aus den vom Transceiver übermittelten Messdaten und der bekannten Wandstärke des Hohlzylinders die Ausbreitungsgeschwindigkeit der Radarwellen, beziehungsweise den Unterschied der Ausbreitungsgeschwindigkeiten ohne und mit Hohlzylinder 1 im Strahlengang. Aufgrund des Laufzeitunterschieds kann auf die Materialkonstanten des Quarzglases des Hohlzylinders 1 geschlossen werden.

Insbesondere kann bei Hohlzylindern 1 aus Quarzglas mit unbekannten aber unterschiedlichen Fluorkonzentrationen die Konzentration dieses Dotierstoffs anhand der obigen Gleichungen (1) und (2) ermittelt werden, sofern alle anderen Materialkonstanten mit Einfluss auf die Ausbreitungsgeschwindigkeit der Radarwellen unverändert sind.

Durch Rotation der Radarquelle 5 um die Längsachse 6 wird ein für unterschiedliche azimutale Richtungen (Winkel) eine gemittelte Brechzahl erhalten, also insgesamt ein azimutales Profil der Ausbreitungsgeschwindigkeit an jeder axialen Position der Radarquelle 5, das sich in ein azimutales Brechzahlprofil bei der Messwellenlänge umrechnen lässt.

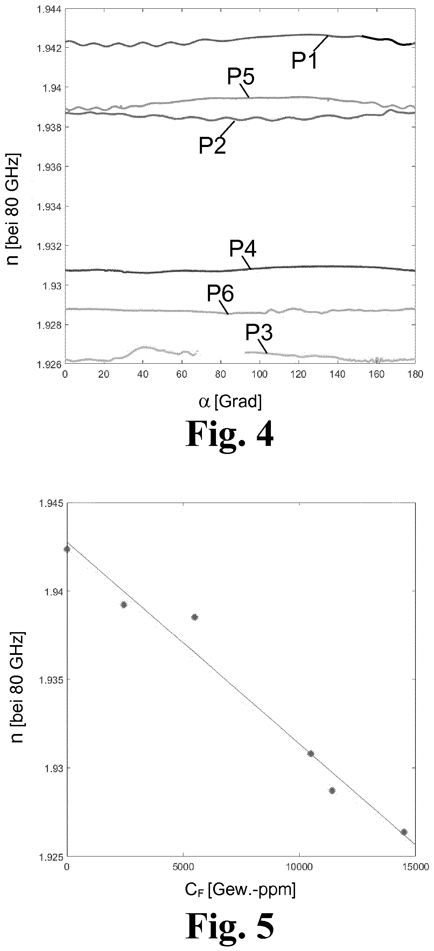

Das Diagramm von **Figur 4** zeigt so ermittelte azimutale Brechzahlprofil, die auf diese Weise an sechs Hohlzylindern 1 aus Quarzglas mit unterschiedlicher Fluor-Dotierung ermittelt worden sind (die Aufnahmen stammen jeweils von etwa der Mitte der jeweiligen Hohlzylinder). Auf der Ordinate ist die Brechzahl n aufgetragen gegen den Einstrahlwinkel $\alpha$ (in Winkelgrade). Demnach zeigen alle Hohlzylinder P1 bis P6 ein etwa gleichmäßiges azimutales Brechzahlprofil auf unterschiedlichem Niveau. In der Spalte 3 von Tabelle 1 sind die jeweiligen Brechzahl-Mittelwerte genannt.

Die Fluor-Konzentrationen $C_F$ der Proben P1 bis P6 wurden auf Basis der eingangs beschriebenen Analysemethode unter Einsatz von Ultraschallwellen ermittelt. Im Diagramm von **Figur 5** sind die Mittelwerte der Brechzahl n gegen die Fluor-Konzentrationen $C_F$ (in Gew.-ppm) aufgetragen. Daraus ist ersichtlich, dass es zwischen der Fluor-Konzentrationen $C_F$ und der Brechzahl einen näherungsweise linearen Zusammenhang gibt. In Spalte 2 von Tabelle 1 sind die jeweiligen gemessenen Fluor-Konzentrationen $C_F$ genannt. Somit kann aufgrund einer einfachen Eichmessung jedem Brechzahl- Mittelwert eine spezifische Fluor-Konzentration zugeordnet werden.

**Tabelle 1**

| Probe | $C_F$ [Gew.-ppm] | n (bei 80 GHz) |
|-------|------------------|----------------|
| P1 | 0 | 1,9523 |
| P2 | 5.500 | 1,9485 |

(fortgesetzt)

| Probe | $C_F$ [Gew.-ppm] | n (bei 80 GHz) |
|---|---|---|
| P3 | 14.500 | 1,9415 |
| P4 | 10.500 | 1,9445 |
| P5 | 2.440 | 1,9511 |
| P6 | 11.420 | 1,9438 |

Durch Translation der der Radarquelle 5 entlang der Hohlzylinder-Längsachse 6 in Richtung des Richtungspfeiles 10 wird ein axiales Profil der Fluorkonzentration im Hohlzylinder 1 erhalten. Die Probenauflagen 2 werden dabei so angesteuert, dass sie von der Stützposition in die Freigabeposition bewegt werden, sobald die Radarquelle 5 ihre Position passiert.

**Patentansprüche**

1. Verfahren zur Bestimmung der Konzentration einer Zusatzkomponente in einem Körper aus glasigem Werkstoff, umfassend die Messung der Differenz zwischen der optischen Weglänge oder der Signallaufzeit einer den Körper aus glasigem Werkstoff in einer Messrichtung durchdringenden Messwelle und der optischen Weglänge oder der Signallaufzeit einer Vergleichsmesswelle, die den Körper aus glasigem Werkstoff nicht durchdringt, **dadurch gekennzeichnet, dass** als Messwelle modulierte Gigahertz-Strahlung eingesetzt wird, die den Frequenzbereich zwischen 20 bis 300 Gigahertz umfasst, dass der Körper aus einem hochkieselsäurehaltigen Glas besteht, und dass die Zusatzkomponente ausgewählt ist aus der Gruppe bestehend aus Hydroxylgruppen, Wasserstoff, ein Metalloxid und/oder ein Halogen.

2. Verfahren nach Anspruch 1, wobei der Körper aus Quarzglas besteht, wobei die Zusatzkomponente Fluor ist.

3. Verfahren nach Anspruch 1 oder 2, wobei die Gigahertz-Strahlung frequenzmoduliert wird.

4. Verfahren nach einem der vorhergehenden Ansprüche, wobei der Körper aus glasigem Werkstoff aus unterschiedlichen Messrichtungen von der Gigahertz-Strahlung durchdrungen wird, wobei für jede Messrichtung eine jeweilige optische Weglänge oder eine jeweilige Signallaufzeit ermittelt wird.

5. Verfahren nach Anspruch 4, wobei der Körper aus glasigem Werkstoff ortsfest in Bezug auf mindestens einen Sender für die Gigahertz-Strahlung positioniert ist, wobei der Sender zur Ermittlung eines dreidimensionalen Profils der Zusatzkomponenten-Konzentration um den Körper aus glasigem Werkstoff bewegt wird.

6. Verfahren nach Anspruch 5, wobei der Abstand zwischen der Oberfläche des Körpers aus glasigem Werkstoff und dem Sender beziehungsweise einem Empfänger größer oder kleiner ist als die in der Hauptabstrahlrichtung der Gigahertz-Strahlung liegende Abmessung des Körpers.

**Claims**

1. A method for determining the concentration of an additional component in a body made of glassy material, comprising measuring the difference between the optical path length or the signal propagation time of a measuring wave penetrating the body made of glassy material in a measuring direction and the optical path length or the signal propagation time of a comparison measuring wave which does not penetrate the body made of glassy material, **characterized in that** modulated gigahertz radiation is used as the measuring wave, which covers the frequency range between 20 and 300 gigahertz, **in that** the body consists of a glass with a high silicic acid content, **and in that** the additional component is selected from the group consisting of hydroxyl groups, hydrogen, a metal oxide and/or a halogen.

2. The method according to claim 1, wherein the body is made of quartz glass, wherein the additional component is fluorine.

3. The method according to claim 1 or 2, wherein the gigahertz radiation is frequency modulated.

4. The method according to one of the preceding claims, wherein the body made of glassy material is penetrated by the gigahertz radiation from different measuring directions, wherein a relevant optical path length or a relevant signal propagation time is determined for each measuring direction.

5. The method according to claim 4, wherein the body made of glassy material is stationary in relation to at least one transmitter for the gigahertz radiation, wherein the transmitter is moved around the body made of glassy material to determine a three-dimensional profile of the additional component concentration.

6. The method according to claim 5, wherein the distance between the surface of the body made of glassy material and the transmitter or a receiver is greater or smaller than the dimension of the body lying in the main radiation direction of the gigahertz radiation.

**Revendications**

1. Procédé permettant de déterminer la concentration d'un composant supplémentaire dans un corps constitué d'un matériau vitreux, comprenant la mesure de la différence entre la longueur de trajet optique ou le temps de propagation de signal d'une onde de mesure traversant le corps constitué de matériau vitreux dans une direction de mesure et la longueur de trajet optique ou le temps de propagation de signal d'une onde de mesure comparative qui ne traverse pas le corps constitué de matériau vitreux, **caractérisé en ce qu'**un rayonnement en gigahertz modulé est utilisé comme onde de mesure, lequel comprend le domaine fréquentiel compris entre 20 et 300 gigahertz, **en ce que** le corps est constitué d'un verre à haute teneur en acide silicique, **et en ce que** le composant supplémentaire est choisi dans le groupe constitué de groupes hydroxyle, hydrogène, oxyde métallique et/ou halogène.

2. Procédé selon la revendication 1, dans lequel le corps est constitué de verre de quartz, dans lequel le composant supplémentaire est du fluor.

3. Procédé selon la revendication 1 ou 2, dans lequel le rayonnement en gigahertz est modulé en fréquence.

4. Procédé selon l'une des revendications précédentes, dans lequel le corps constitué de matériau vitreux est traversé par le rayonnement en gigahertz à partir de différentes directions de mesure, dans lequel une longueur de trajet optique respective ou un temps de propagation de signal respectif est défini pour chaque direction de mesure.

5. Procédé selon la revendication 4, dans lequel le corps constitué de matériau vitreux est positionné de manière fixe par rapport à au moins un émetteur de rayonnement en gigahertz, dans lequel l'émetteur est déplacé autour du corps constitué de matériau vitreux pour la détermination d'un profil tridimensionnel de la concentration de composants supplémentaires.

6. Procédé selon la revendication 5, dans lequel la distance entre la surface du corps constitué de matériau vitreux et l'émetteur ou un récepteur est supérieure ou inférieure à la dimension du corps dans la direction principale d'émission du rayonnement en gigahertz.

**Fig. 1**

**Fig. 2**

**Fig. 3**

EP 3 492 911 B1

Fig. 4

Fig. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2017031006 A1

- DE 102013225306 A1

**In der Beschreibung aufgeführte Nicht-Patentliteratur**

- **FACHARTIKEL VON WEI** ; **TING-CUN**. Acoustic properties of silica glass doped with fluorine. *Journal of Non-Crystalline Solids*, 2003, vol. 321, 126-133